# EUROPEAN PATENT SPECIFICATION

(11) **EP 3 016 581 B1**
(45) Date of publication and mention of the grant of the patent: **15.05.2019**
(21) Application number: 14735673.7
(22) Date of filing: 30.06.2014
(51) Int. Cl.: A61B 5/022, A61B 5/00

(54) **AN IMPROVED SPHYGMOMANOMETER CAPABLE OF DISPLAYING THE QUALITY OF BLOOD PRESSURE READINGS**
VERBESSERTE BLUTDRUCKMESSVORRICHTUNG MIT ANZEIGE DER QUALITÄT VON BLUTDRUCKMESSUNGEN
SPHYGMOMANOMÈTRE AMÉLIORÉ PERMETTANT D'AFFICHER LA QUALITÉ DES MESURES DE PRESSION SANGUINE

(30) Priority: 01.07.2013 GB 201311757
(43) Date of publication of application: 11.05.2016
(73) Proprietor: University of Newcastle Upon Tyne, Newcastle upon Tyne Tyne and Wear NE1 7RU (GB)
(72) Inventor: MURRAY, Alan, Newcastle upon Tyne Tyne and Wear NE1 7RU (GB); ZHENG, Dingchang, Newcastle upon Tyne Tyne and Wear NE1 7RU (GB)
(74) Representative: Boakes, Jason Carrington
(86) International application number: PCT/GB2014/051982
(87) International publication number: WO 2015/001324

(56) References cited:
- EP-A1- 1 077 042
- US-A1- 2007 118 036
- US-A1- 2010 174 202

## Description

The present invention relates to an improved non-invasive sphygmomanometer which is able to calculate the quality of a blood pressure (BP) reading and display quality information to the user. More specifically the invention relates to a sphygmomanometer which uses an inflatable cuff to measure BP and which calculates the quality of the readings, and a quality indicator device for use with the same.

High blood pressure (BP) is one of the leading cardiovascular risk factors for coronary artery disease, congestive heart failure, renal disease and stroke. Despite the importance of BP measurement and its very widespread use, it is generally accepted that it is one of the most poorly performed diagnostic measurements in clinical practice (from the American Heart Association (AHA), and British and European Hypertension Societies (BHS, ESH)). The most noted comment from clinicians and nurses is that consecutive manual BP measurements in the same individual vary significantly. Previous studies have shown that auscultatory BP is affected by different measurement conditions, and a consecutive manual BP difference of 10 mmHg could be easily obtained from the same subject if the measurement conditions are not well controlled, explaining why erroneous readings are regularly obtained, particularly as the aim is to be accurate to 2 mmHg, as indicated on BP measurement displays. However, a single BP measurement is often used to determine treatment, in spite of high variability between measurements.

Sphygmomanometers or blood pressure monitors are well known in the art. Typically they comprise an inflatable cuff, most commonly for positioning around a patient's upper arm at approximately heart height (although in some cases they can be positioned around a patient's wrist or finger), a pressure gauge or transducer for measuring cuff pressure readings and a mechanism for inflating the cuff to restrict blood flow. There is also a valve to allow deflation of the cuff.

Traditional sphygmomanometers are manual and used by trained practitioners to measure and determine the blood pressure of a patient. These devices use a stethoscope for auscultation and require significant training. They must be used in quiet surroundings to allow the practitioner taking the reading to hear the characteristic sounds. Mercury sphygmomanometers of this type are considered to be the "gold standard" for sphygmomanometers providing the most accurate and reproducible blood pressure measurements.

More recently, automated electronic or digital sphygmomanometers have been developed and are commonly used both in doctors' surgeries, hospitals and at home by patients. Automated devices use electronic calculation of oscillometric measurements to determine BP rather than auscultation and as such can be used without significant training, unlike manual sphygmomanometers. They can also be used in a greater range of environments as it is not necessary for the environment to be quiet to obtain the reading.

Automated devices, which utilise the oscillometric technique, measure and calculate systolic and diastolic pressure from the measured empirical oscillometric parameters and the estimated mean arterial pressure. When blood pumps through the arteries of a patient this causes the arteries to flex and pulse. The flexing is due to pressure variations and these variations will pass from the arteries through the arm of the patient (or in some cases wrist or finger) and into an associated pressurised cuff where, although they are small, they can be sensed by a pressure transducer or gauge. The pulses at various cuff pressures, often referred to as complexes, have peak to peak pressure changes which are minimal when the cuff pressure itself is either above systolic pressure or below diastolic pressure, whist the amplitude of the complexes rises to a maximum value when the cuff pressure reaches the mean arterial pressure. The amplitudes of those cuff pressure complexes equivalent to systolic and diastolic pressures have an approximately fixed relationship to the maximum value. The oscillometric method therefore uses amplitude measurements of detected complexes at various cuff pressures. In some cases the cuff pressures are increased in increments until the required readings are obtained, whilst in other cases the cuff is first taken to a high pressure then decreased in increments until the required readings are obtained. It is also possible to provide a smooth rather than incremental pressure reduction. These techniques and the associated algorithms for determining mean arterial pressure, systolic pressure and diastolic pressure are well known in the art.

Although automated digital sphygmomanometers have significant ease of use benefits when compared to "gold standard" mercury sphygmomanometers, it has been well documented that there are problems with the accuracy and reproducibility of blood pressure readings taken with digital sphygmomanometers. In fact, even the gold standard sphygmomanometers can themselves be inaccurate if the quality of the data obtained is less than ideal. This is a real concern as it may lead to patients either incorrectly being given medication that they do not require or having medication withheld in a case where it would be beneficial.

It is well known in the art that the quality of blood pressure measurement can be affected by many things regardless of whether the device used is manual or automated. For example, incorrect cuff placement or patients moving or talking whilst the measurements are being taken can seriously impact the quality of the reading leading to artefacts being superimposed on the oscillation signal and an incorrect blood pressure measurement being obtained. Currently it is very difficult for a user to determine whether a good quality reading, i.e. an accurate reading has been taken.

A number of prior art devices and methods attempt to identify and remove any artefacts from the signal so as not to distort the calculations; however it has been found that this does not work particularly effectively, particularly in older or very ill patients. Alternatively, other devices are known (e.g. from EP 1 077 042) which analyse the signal to give an indication on the quality of the signal, and giving warnings if the signal comprises significant artefacts.

It is an object of the present invention to obviate or mitigate one or more of the problems associated with both manual and automated sphygmomanometers. Throughout this document reference to an automated sphygmomanometer relates to both fully automatic devices where the inflation and deflation of the cuff is electronically controlled e.g. by an electronically operated pump and valve, and to semi-automatic devices where the cuff is inflated by hand using a pumping ball.

The terms oscillometric envelope, envelope of oscillometric pressure or envelope of oscillometric data refer to the amplitudes of oscillations verses the instantaneous pressure in the blood pressure cuff. For healthy patients with a constant reduction in cuff pressure, the shape of the oscillometric envelope can be Gaussian; however this can change, for example stiffened arteries may flatten the curve.

According to a first aspect of the present invention there is provided a sphygmomanometer quality indicator for manual or automated devices operable to evaluate oscillometric pressure data obtained from a sphygmomanometer and to determine the quality of said data, said indicator comprising
- a means for obtaining oscillometric pressure data
- a means for determining the peak to peak amplitude of each pulse to provide an envelope of oscillometric pressure data
- a means for identifying the top of the envelope of oscillometric pressure data
- a means for normalising the pulse amplitudes associated with the envelope of oscillometric pressure data
- a means for identifying the data points leading up to the top of the envelope of oscillometric pressure data
- a means for fitting the identified points to a curve with smooth increasing and decreasing gradients
- a means for calculating the error for at least some of the empirical readings associated with the identified data points
- a means for expressing the result directly as a number or by comparing the error to one or more predetermined ranges to determine the quality of the data, wherein
the curve with smooth increasing and decreasing gradients is a polynomial curve and wherein the quality indicator further comprises a means for computing and including a plurality of theoretical end points, before the points are fitted to the polynomial curve.

Advantageously, the inventors have quantified the relationship between blood pressure variability and the characteristics of the small pressure pulse waveform of the oscillometric envelop that is detected by the pressure gauge associated with the cuff of a sphygmomanometer. They are then able to calculate the quality of the data and express the quality to the user. This will allow the user to make a decision whether to obtain a repeat or whether the quality is sufficient for the particular situation.

A further advantage is that by fitting the data points to a curve rather than overlaying a predetermined curve this allows for variance in how the reading is obtained, allowing for readings being taken over incremental cuff pressure increases or decreases or smooth pressure increases or decreases and also for variance between patients.

Preferably the identification of the data points leading up to the top of the envelope of oscillometric pressure data identifies points up to 80% of the maximum. Advantageously by identifying points up to 80% of the maximum this allows for curve fitting to then be carried out without the peak of the curve distorting or "pulling" the data.

The benefit of including theoretical lower points at an approximation of high cuff pressure is that a flattened start can be artificially included which improves the accuracy of fitting of a polynomial curve to the points.

Preferably between two and thirty theoretical points are added to the envelope at the high cuff pressure.

Most preferably the order of the polynomial curve is 3^{rd} order or higher. 4^{th} order is preferred to obtain an appropriate quality index without forcing the curve to fit to poor quality variations that would come from a higher order curve.

Preferably the quality indicator is adapted to work with existing sphygmomanometers.

Optionally the quality indicator device is associated with a visual or audible quality display which expresses to the user the quality of the data as determined by the quality indicator device.

According to a second aspect of the present invention there is provided a sphygmomanometer operable to evaluate oscillometric pressure data from the sphygmomanometer being attachable to an inflatable/deflatable cuff and attachable inflating apparatus selectively able to apply fluid to the cuff to pressurise it; the sphygmomanometer, comprising;
a pressure sensor able to record cuff pressure and variances therein;
a quality indicator device as in the first aspect programmed to evaluate oscillometric pressure data and cuff pressure data obtained from the pressure sensor and to determine the quality of said data by
- determining from the oscillometric pressure data the peak to peak amplitude of each pulse to provide an envelope of oscillometric pressure data
- identifying the top of the envelope of oscillometric pressure data
- normalising the pulse amplitudes associated with the envelope of oscillometric pressure data
- identifying the data points leading up to the top of the envelope of oscillometric pressure data
- fitting a plurality of the identified points with smooth increasing and decreasing gradients
- calculating the error for at least some of the empirical readings associated with the identified data points
- expressing the result.

The result may be displayed directly as a number or the error may be compared to one or more predetermined ranges to determine the quality of the data

Preferably the sphygmomanometer is provided with an inflatable/deflatable cuff which has a pressure sensor associated therewith which is able to sense and record cuff pressure and variances therein.

Preferably the sphygmomanometer is provided with inflating apparatus selectively able to apply fluid to an associated cuff to pressurise it.

Preferably the blood pressure reading is displayed visually.

This can be by a digital display or via a scale and moveable indicator.

The quality indicator is achieved using a microprocessor.

Preferably the quality of the reading is expressed to the user using a visual quality display.

Optionally the visual quality display is associated with the blood pressure display.

Optionally the visual quality display comprises a number of colour coded LED lights.

Most preferably the visual quality display comprises a linear array of up to nine colour coded LED lights which show up to nine different qualities

It would be clear to a skilled person that a number of display types could be used, e.g. lights, digital displays etc. to show the user the quality of the data and allow them to make a decision as to whether additional BP readings are required. Optionally the quality indicator device is programmed to automatically retake blood pressure readings when the quality is determined to be below a certain threshold.

The present invention may be used in a method, which uses oscillometric data, of determining the quality of a blood pressure reading from a sphygmomanometer comprising;
- obtaining an envelope of oscillometric data;
- identifying the top of the envelope of oscillometric pressure data
- normalising the pulse amplitudes associated with the envelope of oscillometric pressure data
- identifying the data points leading up to the top of the envelope of oscillometric pressure data
- fitting a plurality of the identified points with smooth increasing and decreasing gradients
- calculating the error for at least some of the empirical readings associated with the identified data points
- expressing the result.

The result may be displayed directly as a number or the error may be compared to one or more predetermined ranges to determine the quality of the data Preferably identification of the top of the envelope of oscillometric pressure data identifies points up to 80% of the maximum.

Preferably between two and thirty theoretical points are added to the envelope at the high cuff pressure.

Preferably a polynomial curve is fitted to the data points.

Optionally the method of the third aspect is carried out by a computer program.

The preceding discussion of the background to the invention is intended only to facilitate an understanding of the present invention.

In order to provide a better understanding of the present invention, embodiments will now be described with reference to the following figures in which;
Figure 1 shows an illustration of the normalised amplitude of the extracted oscillometric envelope data and the calculation of quality index from the recorded oscillometric waveform; and

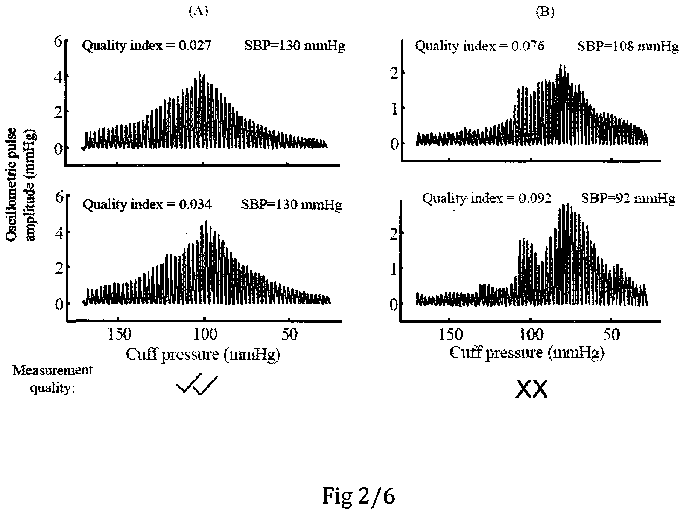

Figure 2 shows examples of the recorded oscillometric waveforms with good quality index (A, left two sub-figures) and poor quality index (B, right two sub-figures). Small measurement error (0 mmHg) was obtained between two measurements with good quality indices, however, the poor oscillometric waveforms were associated with a large measurement error (16 mmHg); and
Figure 3 shows examples of waveforms from which (A) an accurate BP reading may be obtained and (B) an inaccurate BP reading may be obtained; and
Figure 4 shows an example waveform from a patient with atrial fibrillation (AF) where a curve has been fitted in accordance with the present invention to give a poor quality index of 0.1336; and
Figure 5: shows an example waveform from a patient with frequent ectopic beats where a curve has been fitted in accordance with the present invention to give a poor quality index of 0.1472; and
Figure 6 shows a schematic of the LED display

It can be noted that these rhythms can be identified due to specific variations in pulse amplitudes.

Throughout the description and claims of this specification, the singular encompasses the plural unless the context otherwise requires. In particular, where the indefinite article is used, the specification is to be understood as contemplating plurality as well as singularity, unless the context requires otherwise.

Features, integers or characteristics, compounds described in conjunction with a particular aspect, embodiment or example of the invention are to be understood to be applicable to any other aspect, embodiment or example described herein unless incompatible therewith. In particular, it will be fully understood that alternative inflation options for the cuff are available and that inflation and deflation may be fully automated or may be semi-automated with cuff inflation being via a hand operated bulb pump. It will also be understood that although the examples reference a cuff positioned around a patient's upper arm, any alternative cuff which is able to detect and utilise oscillometric data can be used; the cuff could be adapted for use on other appropriate body parts which allow oscillometric pressure readings to be obtained e.g. the wrist, finger or other appropriate part of a patient.

### Evidence of the relationship between oscillometric waveform quality and BP measurement error

The inventors carried out a study to determine whether a relationship existed between oscillometric waveform quality and BP measurement error. Thirty healthy subjects were studied, with details of the subjects shown in table 1.

**Table 1. General data information for the subjects studied.**

| **Subject information** | | |
|---|---|---|
| No. subjects | 30 | |
| No. male | 19 | |
| No. female | 11 | |
| | | |

| | **Mean** | **SD** |
|---|---|---|
| Age (years) | 45 | 12 |
| Height (cm) | 172 | 7 |
| Weight (kg) | 76 | 10 |
| Arm circumference (cm) | 29 | 2 |

For each subject, there were four identical sessions where BP measurements were taken, separated by a time interval of 3-4 min. The first session was regarded as a trial session to introduce the measurement protocol to the subjects, and was excluded from further data analysis. Within each session, three BP measurements were respectively performed under three different measurement conditions, with a one minute rest interval between each. The first measurement was under relaxing conditions by advising the subjects to close their eyes and breathe smoothly, and the second one under normal condition with their eyes open. For the third condition, subjects were asked to breathe more deeply and regularly. To simplify the description, the three measurement conditions are referred to as 'Relaxing', 'Normal' and 'Deep Breathing'. The conditions were designed to induce BP measurement variability. In total, 270 measurements were taken from all 30 subjects, with 9 measurements for each subject being used.

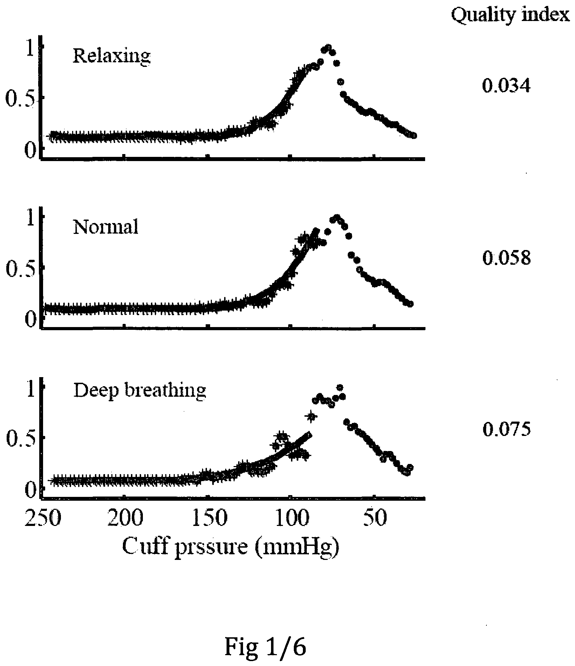

The oscillometric pulses were extracted from the recorded cuff pressure after segmenting each pulse and removing the baseline cuff pressure. The segmentation borders were at the feet of oscillometric pulses. Manual check was also performed to ensure the correct pulse feet were identified after the segmentation procedure. The peak of each oscillometric pulse was then identified with its amplitude measured. For each recording, all the oscillometric pulses were then normalised to the maximum oscillometric pulse. Fig. 1 shows some examples of the normalised amplitude of the extracted oscillometric pulses as the cuff pressure was reduced.

Thirty pulses were artificially added before the first recorded oscillometric peak to construct a waveform with flat feature at the very high pressure region. These added pulses had the same peak amplitude as the first oscillometric pulse, and the interval between any two consecutive peaks were the same, which was referred from the value between the first and second recorded oscillometric pulses.

A 4th order polynomial curve was then used to fit the added peaks and these recorded oscillometric pulse peaks with their amplitudes no more than 80% of the maximum oscillometric pulse amplitude. Fig. 1 also illustrates the fitted polynomial curves for three example waveforms. Next, Root Mean Square Error (RMSE) was calculated to quantify the difference between the values on the fitted polynomial curve and the peak amplitudes actually recorded, and was defined as the oscillometric waveform quality index.

In order to investigate the relationship between the oscillometric waveform quality and measurement error (BP difference from two measurements), any possible pairs of quality indices and BPs (SBP and DBP) between the three measurement conditions within the same measurement session were used for each subject. In total, there were 270 pairs (from 30 subjects, 3 repeated measurement sessions, and 3 pairs for each repeat). After excluding a few noisy recording, 266 pairs were left for final analysis.

Depending on the bigger value from the two quality indices in a pair, each pair was classified to one of the 4 quality bands (best quality; good quality; poor quality; poorest quality). They were simply represented with symbols of √√, √, × and ××, and they were determined using the following criteria:
√√--Best quality: both quality indices in a pair <=0.035;
√--Good quality: 0.035< bigger value from the two quality indices in a pair <= 0.05;
×--Bad quality: 0.05< bigger value from the two quality indices in a pair <= 0.07;
××--Poorest quality: bigger value from the two quality indices in a pair > 0.07.

In this particular implementation a higher index indicates poorer quality. Here, the size of the units are very small because they are actual measurement values.
The overall mean and standard error of the mean (SEM) for SBP, DBP and Quality Index were calculated from all the subjects separately for each measurement condition. Their values under the normal and deep breathing conditions were compared with those from the relaxing condition, with the mean differences calculated. All differences were paired values in each subject.

Next, the overall mean and SD of the absolute SBP and DBP measurement error were calculated from all the pairs separately for each quality band. The percentage of measurement with a high error (absolute SBP and DBP difference from a pair of measurement >10 and 8 mmHg, respectively) was also obtained for each quality band.

Using the SPSS Statistics 17 software package (SPSS Inc., USA), Repeated Measures Analysis of Variance was performed to study the measurement repeatability and the effect of measurement condition on SBP, DBP and Quality Index. In this analysis, each subject was his or her own control. The post-hoc Fisher's least significant difference (LSD) test was used to make individual comparison between means. A P value below 0.05 was considered statistically significant.

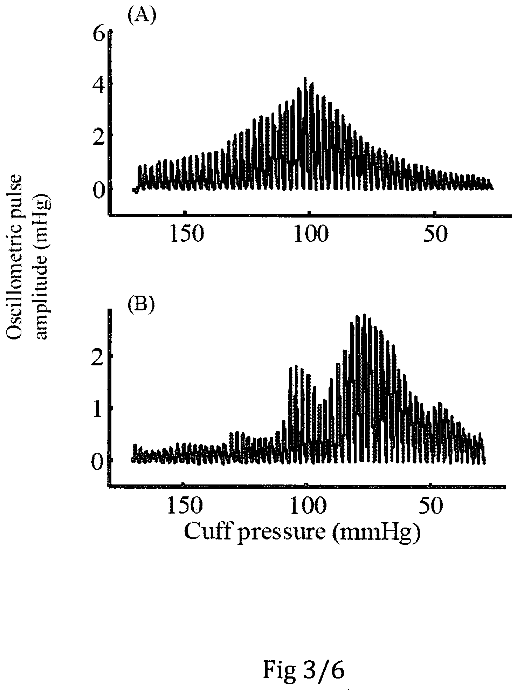

From this study it has been possible for the inventors to identify that there is a correlation between the repeatability of a blood pressure reading and the quality of the envelope of oscillation data, or quality of the oscillometric waveform, obtained during the reading. Figure 3 shows examples of envelopes of oscillation data obtained from a sphygmomanometer which would provide (A) a high quality accurate reading or (B) a low quality inaccurate reading.

### Improved sphygmomanometer

A device according to one aspect of the present invention is generally a sphygmomanometer which has a cuff which in use is placed around a patient's upper arm at approximately heart height. The cuff comprises a transducer or pressure gauge (not shown) which is able to detect cuff pressure and variances therein. A hand operated bulb with an air control valve is in communication with the cuff (for example linked via coiled connector tubing) and can be used to inflate the cuff by introducing air into the cuff and increasing the pressure therein. The cuff also has a release valve (not shown) which allows the cuff 2 to be deflated and thus the pressure therein decreased. The cuff is adapted to automatically deflate from approximately 200 to 10 mmHg at a rate of 2-3 mmHg/s. The cuff is also in communication with a pressure display which displays the pressure readings from the cuff 2. This pressure display is shown as a scale and pointer but it could for example be a digital display. The cuff is also in communication with a quality indicator device, described in more detail below, and a display.

### Quality indicator device

The device 1 also contains a control microprocessor 5 which is able to digitally capture and record the pressure readings obtained from the pressure gauge. The data is captured at a sample rate of 2000Hz.

The device is programmed to first extract and normalise the oscillometric pulse data. The oscillometric pulses are extracted from the recorded cuff pressure after segmenting each pulse and removing the baseline cuff pressure The segmentation borders are taken as being at the feet of oscillometric pulses. The peak of each oscillometric pulse is then identified and its amplitude measured. All of the oscillometric pulses are then normalised to the maximum oscillometric pulse.

In order to improve the curve fitting, a preferred version of the quality indicator device is programmed to artificially add pulses to result in a waveform construct with a flattened base. Thirty pulses are artificially added before the first recorded oscillometric peak to construct a waveform with flat feature at the very high pressure region. It will be appreciated that an alternative number of artificial pulses could be included. These added pulses have the same peak amplitude as the first oscillometric pulse, and the interval between any two consecutive peaks is the same, which was referred from the value between the first and second empirically recorded oscillometric pulses.

A 3^{rd} or 4th order polynomial curve is then used to fit the added peaks and the recorded oscillometric pulse peaks.

Root Mean Square Error (RMSE) is then calculated to quantify the difference between the values on the fitted polynomial curve and the empirical peak amplitudes actually recorded, (for each oscillation the systolic BP and the diastolic BP are recorded) to give an oscillometric waveform quality index.

In one embodiment the quality is classified into four groups depending on the quality indices;
Best quality: quality indices <=0.035;
Good quality: 0.035< quality indices <= 0.05;
Bad quality: 0.05< quality indices <= 0.07;
Poorest quality: quality indices > 0.07.

It would be clearly appreciated that fewer or additional predetermined ranges of error values could be included to allow alternative classification of the data. In a particularly preferred embodiment there are nine predetermined ranges or classes into which the error value may fall.

In particular, a device with 9 LEDs is envisaged where each LED relates to a predetermined range. LEDs may be equally arranged with the quality index from 0 to 1, with the range of 0.125 for each. It would be understood that the number and extent of the ranges could easily be altered.

### Display

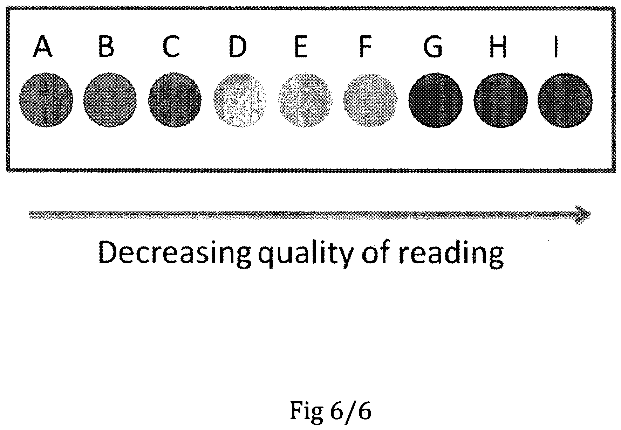

The results of the quality index are then expressed to the user. This may be by any appropriate mechanism, however it is envisaged that a visual representation would be used. A preferred option would be to provide an array of nine LEDs in a linear formation (Fig 6). These would be arranged as three green LEDs (A, B and C), followed by three amber LEDs (D, E and F) followed by three red LEDs (G, H and I). Although the colours are not shown clearly in the figures, it would be appreciated by a skilled user that any colour combination could be used. The quality indicator device operates the LED array and will express the quality data by turning on an appropriate light in response to the quality of the reading. Higher quality of the data (which will occur when there are minimal deviations between the empirically obtained data points and the fitted curve) will turn on one of the green LEDs where LED A indicates the highest quality data, LED B the next best and LED C the third best quality. The lowest poor quality readings being visually expressed as a single red LED turning on, where LED I indicates the poorest quality data. Mid quality readings will illuminate one of the three amber LEDs D, E or F, LED D indicating better quality data than F.. This will allow a user a very clear visual representation of the quality of the blood pressure reading that is taken. They can then decide on the basis of the quality reading whether to take a second blood pressure measurement or whether it is fit for purpose (it will be appreciated that there are some situations where a very accurate reading is essential, whilst in other cases some margin of error will be accepted).

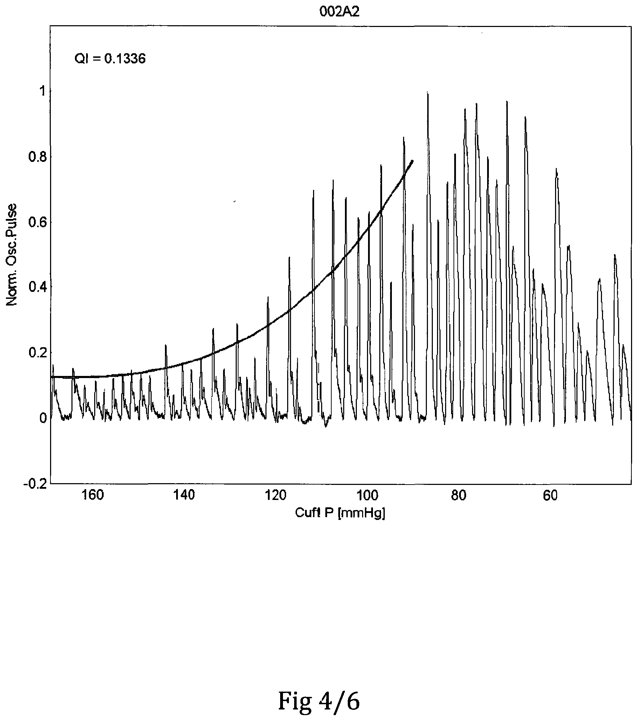

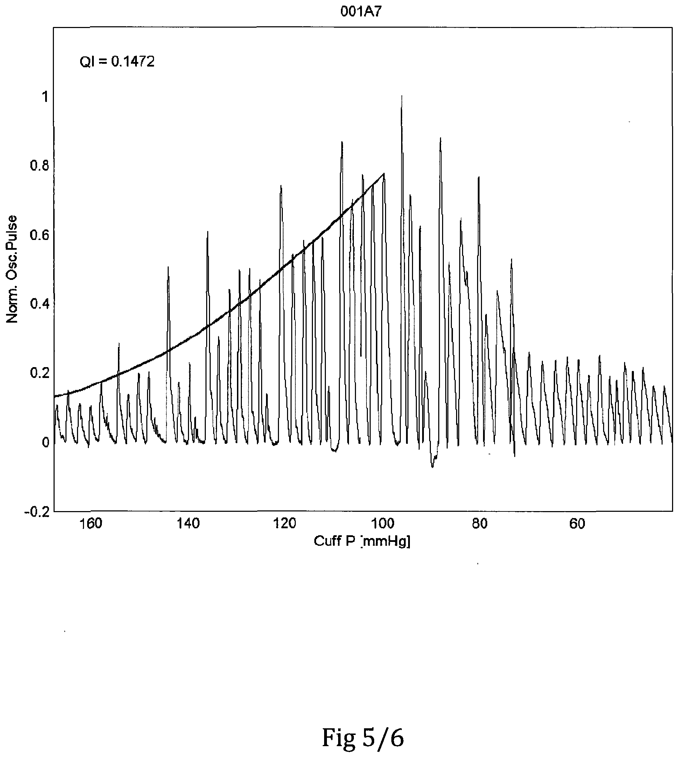

It has been noted by the inventors that there is a particular benefit with the present invention when patients have irregular heartbeats. Figures 4 and 5 show oscillometric waveform data from patients with atrial fibrillation (AF) (Figure 4) and ectopic beats (Figure 5). It can be seen that in each case a relatively large quality index value is obtained which is can indicate an abnormality which may not be otherwise be recognised.

It is also envisaged that the use of sphygmomanometers including the quality indicator device of the present invention would be very useful in training situations.

## Claims

1. A sphygmomanometer quality indicator operable to evaluate oscillometric pressure data obtained from a manual or automated sphygmomanometer and to determine the quality of said data, said indicator comprising:
- a means for obtaining oscillometric pressure data
- a means for determining the amplitude of each pulse to provide an envelope of oscillometric pressure data
- a means for identifying the top of the envelope of oscillometric pressure data
- a means for normalising the pulse amplitudes associated with the envelope of oscillometric pressure data
- a means for identifying the data points leading up to the top of the envelope of oscillometric pressure data
- a means for fitting the identified points to a curve with smooth increasing and decreasing gradients
- a means for calculating the error for at least some of the empirical readings associated with the identified data points; and
- a means for expressing the result directly as a number or by comparing the error to one or more predetermined ranges to determine the quality of the data; wherein
the curve with smooth increasing and decreasing gradients is a polynomial curve; and
wherein the quality indicator further comprises a means for computing and including a plurality of theoretical lower end points, before the points are fitted to the polynomial curve.

2. A sphygmomanometer quality indicator as in any of the previous claims wherein the quality indicator device is in communication with the cuff of a sphygmomanometer and/or wherein the quality indicator is associated with a quality display which expresses to the user the quality of the data as determined by the quality indicator.

3. A sphygmomanometer operable to evaluate oscillometric pressure data, the sphygmomanometer being attachable to an inflatable/deflatable cuff and attachable to inflating apparatus selectively able to apply fluid to the cuff to pressurise it; the sphygmomanometer comprising;
a pressure sensor able to record cuff pressure and variances therein;
a quality indicator device as in claim 1 or claim 2;
a quality display which expresses to the user the quality of the data as determined by the quality indicator device; and
a blood pressure display adapted to display blood pressure information.

4. A sphygmomanometer as in claim 3 wherein the quality indicator device is a microprocessor.

5. A sphygmomanometer as in any of claims 3 or 4 wherein the quality display is a visual quality display and/or wherein the visual quality display is associated with the blood pressure display.

6. A sphygmomanometer as in any of claims 3 to 5 wherein the visual quality display comprises a number of colour coded LED lights, preferably a linear array of nine colour coded LED lights.

## Patentansprüche

1. Qualitätsindikator für Blutdruckmessgeräte, der betreibbar ist zum Auswerten von oszillometrischen Druckdaten, die von einem manuellen oder automatisierten Blutdruckmessgerät erhalten wurden, und zum Bestimmen der Qualität der Daten, wobei der Indikator umfasst:
ein Mittel zum Erhalten von oszillometrischen Druckdaten
ein Mittel zum Bestimmen der Amplitude jedes Pulses, um eine Hüllkurve der oszillometrischen Druckdaten bereitzustellen
ein Mittel zum Identifizieren der Spitze der Hüllkurve der oszillometrischen Druckdaten ein Mittel zum Normieren der Pulsamplituden, die der Hüllkurve der oszillometrischen Druckdaten zugeordnet sind
ein Mittel zum Identifizieren der Datenpunkte, die bis zur Spitze der Hüllkurve der oszillometrischen Druckdaten führen
ein Mittel zum Anpassen der identifizierten Punkte an eine Kurve mit gleichmäßig steigenden und fallenden Steigungen
ein Mittel zum Berechnen des Fehlers für mindestens einige der den identifizierten Datenpunkten zugeordneten empirischen Messwerte;
und ein Mittel zum direkten Ausdrücken des Ergebnisses als eine Zahl oder durch Vergleichen des Fehlers mit einem oder mehreren vorbestimmten Bereichen, um die Qualität der Daten zu bestimmen; wobei
die Kurve mit gleichmäßig steigenden und fallenden Gradienten eine Polynomkurve ist;
und wobei der Qualitätsindikator ferner ein Mittel zum Ausrechnen und Einschließen einer Vielzahl von theoretischen unteren Endpunkten umfasst, bevor die Punkte an die Polynomkurve angepasst werden.

2. Qualitätsindikator für Blutdruckmessgeräte nach einem der vorhergehenden Ansprüche, wobei die Qualitätsindikatorvorrichtung mit der Manschette eines Blutdruckmessgeräts in Verbindung steht und/oder wobei der Qualitätsindikator einer Qualitätsanzeige zugeordnet ist, welche gegenüber dem Benutzer die Qualität der Daten ausdrückt, wie durch den Qualitätsindikator bestimmt.

3. Blutdruckmessgerät, das zum Auswerten von oszillometrischen Druckdaten betreibbar ist, wobei das Blutdruckmessgerät an einer aufblasbaren/ablassbaren Manschette befestigbar und an einem Aufblasapparat befestigbar ist, der selektiv in der Lage ist, Flüssigkeit auf die Manschette zu beaufschlagen, um sie unter Druck zu setzen; wobei das Blutdruckmessgerät umfasst;
einen Drucksensor, der in der Lage ist, den Manschettendruck und Abweichungen darin aufzuzeichnen; eine Qualitätsindikatorvorrichtung nach Anspruch 1 oder Anspruch 2;
eine Qualitätsanzeige, welche gegenüber dem Benutzer die Qualität der Daten ausdrückt, wie durch die Qualitätsindikatorvorrichtung bestimmt;
und eine Blutdruckanzeige, die zum Anzeigen von Blutdruckinformationen umgewandelt wurde.

4. Blutdruckmessgerät nach Anspruch 3, wobei die Qualitätsindikatorvorrichtung ein Mikroprozessor ist.

5. Blutdruckmessgerät nach einem der Ansprüche 3 oder 4, wobei die Qualitätsanzeige eine visuelle Qualitätsanzeige ist und/oder wobei die visuelle Qualitätsanzeige der Blutdruckanzeige zugeordnet ist.

6. Blutdruckmessgerät nach einem der Ansprüche 3 bis 5, wobei die visuelle Qualitätsanzeige eine Anzahl von farbcodierten LED-Leuchten umfasst, vorzugsweise eine lineare Reihe von neun farbcodierten LED-Leuchten.

## Revendications

1. Indicateur de la qualité pour sphygmomanomètre servant à évaluer des données de pression oscillométriques obtenues à partie d'un sphygmomanomètre manuel ou automatique et pour déterminer la qualité desdites données, ledit indicateur comprenant :
- un moyen pour obtenir des données de pression oscillométriques
- un moyen pour déterminer l'amplitude de chaque pulsation pour fournir une enveloppe de données de pression oscillométriques
- un moyen pour identifier la partie supérieure de l'enveloppe de données de pression oscillométriques
- un moyen pour normaliser les amplitudes d'impulsions associées à l'enveloppe de données de pression oscillométriques
- un moyen pour identifier les points de données conduisant à la partie supérieure de l'enveloppe de données de pression oscillométriques
- un moyen pour ajuster les points identifiés à une courbe avec des gradients croissants et décroissants réguliers
- un moyen pour calculer l'erreur pour au moins une partie des lectures empiriques associées aux points de données identifiés ; et
- un moyen pour exprimer le résultat directement sous la forme d'un nombre ou en comparant l'erreur à une ou plusieurs plages préfinies pour déterminer la qualité des données ;
ladite courbe avec des gradients croissants et décroissants réguliers étant une courbe polynomiale ; et
ledit indicateur de la qualité comprenant en outre un moyen pour calculer et inclure une pluralité de points d'extrémité inférieurs théoriques, avant ajustement des points à la courbe polynomiale.

2. Indicateur de la qualité pour sphygmomanomètre selon l'une quelconque des revendications précédentes, ledit dispositif indicateur de la qualité étant en communication avec le brassard d'un sphygmomanomètre et/ou ledit indicateur de la qualité étant associé à un écran d'affichage de la qualité qui exprime à l'utilisateur la qualité des données déterminée par l'indicateur de la qualité.

3. Sphygmomanomètre servant à évaluer des données de pression oscillométriques, le sphygmomanomètre pouvant être fixé à un brassard gonflable/dégonflable et pouvant être fixé à un appareil de gonflage pouvant sélectivement appliquer un fluide sur le brassard pour le mettre sous pression ; le sphygmomanomètre comprenant :
un capteur de pression pouvant enregistrer la pression du brassard et les variations de celle-ci ;
un dispositif indicateur de la qualité selon la revendication 1 ou 2 ;
un écran d'affichage de la qualité qui exprime à l'utilisateur la qualité des données déterminée par le dispositif indicateur de la qualité ; et
un écran d'affichage de la pression sanguine conçu pour afficher des informations de pression sanguine.

4. Sphygmomanomètre selon la revendication 3, ledit dispositif indicateur de la qualité étant un microprocesseur.

5. Sphygmomanomètre selon l'une quelconque des revendications 3 ou 4, ledit écran d'affichage de la qualité étant un écran visuel d'affichage de la qualité et/ou ledit écran visuel d'affichage de la qualité étant associé à l'écran d'affichage de la pression sanguine.

6. Sphygmomanomètre selon l'une quelconque des revendications 3 à 5, ledit écran visuel d'affichage de la qualité comprenant un certain nombre de lumières DEL à code couleur, de préférence un réseau linéaire de neuf lumières DEL à code couleur.
